# EUROPÄISCHE PATENTSCHRIFT

(11) **EP 0 654 484 B1**
(45) Veröffentlichungstag und Bekanntmachung des Hinweises auf die Patenterteilung: **23.04.2003**
(21) Anmeldenummer: 94116380.0
(22) Anmeldetag: 18.10.1994
(51) Int. Cl.: C08B 37/16, A61K 47/48, A61K 31/385, A61K 31/19

(54) **Arzneimittelzubereitungen enthaltend Thioctsäure oder Dihydroliponsäure in Form von Einschlussverbindungen mit Cyclodextrinen und in Form von Granulaten, Kau- oder Brausetabletten**
Pharmaceutical compositions containing inclusion compounds of thioctic or dihydroliponic acid with cyclodextrins and in granule form or in chewable or effervescent tablet form
Compositions pharmaceutiques contenant de l'acide thioctique ou dihydroliponic sous forme de composés d'inclusion avec des cyclodextrines et sous forme de granulés, de comprimés à mâcher ou effervescents

(30) Priorität: 11.11.1993 DE 4338508
(43) Veröffentlichungstag der Anmeldung: 24.05.1995
(60) Teilanmeldung: 00121934.4
(73) Patentinhaber: VIATRIS GmbH & Co. KG, 60314 Frankfurt am Main (DE)
(72) Erfinder: Hettche, Helmut, Dr., D-63128 Dietzenbach (DE)
(74) Vertreter: Wibbelmann, Jobst, Dr., Dipl.-Chem.

(56) Entgegenhaltungen:
- EP-A- 0 427 246
- EP-A- 0 427 247
- Römpps Chemie-Lexicon, 8. Auflage, Franckh'sche Verlaghandlung, Stuttgart, 1983, Seiten 2379 und 4238
- "Thiocacid", Gebrauchs- und Fachinformationen, ASTA Pharma AG, Frankfurt

## Beschreibung

Die vorliegende Erfindung betrifft Arzneimittelformulierungen, die Thioctsäure enthalten, wobei die Thioctsäure vorliegt in Form von Granulaten, Kau-, Trink- oder Brausetabletten.

Thioctsäure (alpha-Liponsäure) ist chemisch eine 1,2-Dithiacyclopentan-3-valeriansäure. Die Erfindung bezieht sich nicht nur auf die razemische Form, sondern ebenso auf die reine (R)- beziehungsweise (S)-Thioctsäure sowie auf Gemische aus (R)- und (S)-Thioctsäure mit beliebiger Zusammensetzung.

Thioctsäure ist Bestandteil des Zellstoffwechsels und wird daher in vielen Pflanzen und tierischen Organismen gefunden. Sie wirkt als eines der Coenzyme bei der oxydativen Decarboxylierung von Pyruvat und anderen alpha-Ketosäuren.
Thioctsäure wird seit längerer Zeit bei verschiedenen Erkrankungen eingesetzt, so unter anderem bei Lebererkrankungen (siehe auch Römpps Chemie-Lexikon, 8. Auflage, Franck'sche Verlagshandlung, Stuttgart, 1983, Seiten 2379 und 4238), bei Leberschädigungen durch Pilzvergiftung sowie bei diabetischer und alkoholischer Polyneuropathie, einer mit Stoffwechselerkrankungen einhergehenden Veränderung peripherer Nerven. Enantiomerenrein sind (R)- und (S)-Thioctsäure beispielsweise nach den Vorschriften im EP 261 336 zugänglich.

Die derzeit handelsüblichen thioctsäurehaltigen Tablettenzubereitungen enthalten maximal 600 mg Thioctsäure in einer Filmtablette zu 850 mg.

Solche Tabletten können auch mit einem magensaftresistenten Überzug versehen werden, siehe z. B. EP-A-0 427 247.

Im Anfangsstadium der Behandlung und bei der Behandlung von AIDS werden jedoch hohe Dosierungen von Thioctsäure verabreicht, die bei peroraler Applikation bis zu mehreren Gramm täglich betragen können. Bis zu einer Dosierung von 300 mg Thioctsäure sind Filmtabletten in einer Form herstellbar, die noch einigermaßen schluckbar ist. Bei höheren Dosierungen nehmen die Formlinge jedoch eine Größe an, die sich negativ auf die Patienten-Compliance auswirkt. Derartige Tabletten sind auch im Magen-Darm-Trakt schlecht verträglich und lassen sich somit nicht mehr applizieren.

Erfolglos wurde versucht, das Problem durch die Gabe einer Lösung von Thioctsäure in Form ihrer Salze zu lösen, wie dies bereits in der europäischen Anmeldung 0 427 246 A2 vorgeschlagen wurde, beispielsweise als Lysin-, Arginin-, Ethylendiamin- oder Trometamolsalz. Diese Thioctsäuresalze weisen aber einen unbefriedigenden Geschmack auf.

Ebenfalls erfolglos wurde versucht, das Problem durch Gabe einer Suspension der freien Thioctsäure zu lösen.

Die Thioctsäure zeichnet sich aber durch einen stark brennenden Geschmack aus, der lange anhält. Der Weg der Applikation als Trinksuspension ließ sich daher bisher ebenfalls nicht beschreiten.

Trinklösungen haben darüber hinaus den Nachteil, daß sie in angebrochenen Flaschen nicht stabil sind und infolgedessen in Einzeldosisbehälter abgefüllt werden müssen. Zu diesem Zweck wird die Lösung in Glasampullen eingeschweißt. Die Herstellung der Ampullen ist aufwendig, desgleichen die Verpackung. Die Ampulle muß vom Patienten vor der Anwendung erst geöffnet werden. Dies ist für die orale Anwendung ein umständliches Vorgehen. Zudem hat die Lösung ein relativ hohes Gewicht und beansprucht viel Platz für die Lagerung. Der wesentliche Nachteil der Trinklösung ist aber der sehr unangenehme Geschmack, so daß sie trotz Aromatisierung von Probanden sehr ungern eingenommen wird.

Es besteht also ein dringender Bedarf nach Verfahren, die es erlauben, Thioctsäure in hohen Dosierungen mit guter Patienten-Compliance peroral zu applizieren. Dieses Ziel wird erfindungsgemäß dadurch erreicht, daß Thioctsäure nicht in gelöster oder komprimierter Form, sondern als Granulat oder Kau- oder Trinktablette oder in Form einer in Flüssigkeit aufgelösten Brausetablette appliziert wird.

Gegenstand der Erfindung ist somit ein Granulat oder eine Kau-, Trink- oder Brausetablette, enthaltend als Wirksubstanz Thioctsäure oder deren Salze, die in einem Granulat vorliegen, das mit physiologisch verträglichen Überzügen, die in Wasser und Speichel unlöslich, aber im Magen- oder Intestinalsaft löslich sind, versehen ist. Das Granulat kann entweder direkt eingenommen, mit Nahrung vermischt oder in Flüssigkeit suspendiert bzw. aufgelöst durch Essen oder Trinken appliziert werden.

Die genannten Darreichungsformen haben außerdem den Vorteil der verbesserten Verträglichkeit und Bioverfügbarkeit. Das Granulat kann in Beutel abgefüllt, zu Kautabletten verpreßt oder nach Zufügung einer physiologisch unbedenklichen Brausemischung zu Brausetabletten verpreßt werden.
Es enthält, bezogen auf einen Gewichtsteil Thioctsäure 0.001 - 1 Gewichtsteile Bindemittel 0.001 - 0.1 Gewichtsteile Fließhilfsmittel sowie gegebenenfalls Netzmittel und physiologisch unbedenkliche Geschmacks-, Süß-und/oder Aromastoffe. Im Falle einer Kautablette wird das Granulat mit 0.01 - 0.02 Gewichtsteilen eines Gegenklebemittels und gegebenenfalls weiteren Geschmackskorrigentien wie beispielsweise Fructose, Xylit, Sorbit oder Mannit vermischt und zu Tabletten verpreßt.

Im Falle einer Brausetablette wird das Granulat mit 0.05 - 30 Gewichtsteilen einer üblichen physiologisch unbedenklichen Brausemischung vermengt und nach Zufügen von 0.01 -0.02 Gewichtsteilen eines Gleitmittels, zu Tabletten verpreßt.

Als Salzbildner der Thioctsäure kommen in Frage: Basische Aminosäuren wie Arginin oder Lysin, physiologisch verträgliche Alkali- oder Erdalkalihydroxide-, -carbonate oder hydrogencarbonate, Ammoniumhydroxid, Amine der Formel N R₁R₂R₃ worin die Reste R₁, R₂ und R₃ gleich oder verschieden sind und Wasserstoff, C₁-C₄-Alkyl oder C₁-C₄ Hydroxyalkyl bedeuten wie beispielsweise Mono-und Diethanolamin, 1-Amino-2-propanol, 3-Amino-1-propanol, Alkylendiamine mit einer Alkylenkette aus 2 bis 6 C-Atomen wie Ethylendiamin oder Hexamethylentetramin, gesättigte cyclische Aminoverbindungen mit 4 - 6 Ringkohlenstoffatomen wie Piperidin, Piperazin, Pyrrolidin, Morpholin, N-Methylglucamin, Kreatin, Trometmol, N-Methyl-morpholin. Bevorzugt werden Salze mit Erdalkalimetallen (Calcium-, Magnesiumsalz), basischen Aminosäuren und Trometamol. Die im folgenden angegebenen Mengen für Thioctsäure sind im Falle des Einsatzes der Salze entsprechend dem Molekulargewicht umzurechnen.

Zur peroralen Applikation hoher Dosierungen der Thioctsäure besteht, wie erwähnt, ein dringender Bedarf nach Verfahren, die es erlauben, den brennenden Geschmack der Thioctsäure abzumildern bzw. ganz zu eliminieren.

Enantiomere der Thioctsäure weisen niedrige Schmelzpunkte auf (R-Thioctsäure: 47 °C; S-Thioctsäure: 46 °C). Die Herstellung von schnell zerfallenden Tabletten im industriellen Großmaßstab wird dadurch sehr erschwert, well es bei der Aufbereitung der Wirkstoffe zur Herstellung der Tabletten (z.B. Trocknen des Granulats, Auftragen und Trocknen des Filmes bei Filmcoating) zur Erwärmung der Tabletten und damit zum Sintern des niedrigschmelzenden Wirkstoffes kommen kann. Dies hat Auswirkungen auf die Zerfallszeit der Tabletten und auf die Bioverfügbarkeit des Wirkstoffes.

Ein weiterer Nachteil der enantiomerenreinen Formen der Thioctsäure ist die Tatsache, daß die reine R- und S-Thioctsäure leicht zur Polymerisation neigen.

Gegenstand der Erfindung ist daher die Bereitstellung von Darreichungsformen der Thioctsäure (R-Enantiomer oder S-Enantiomer oder Racemat) mit der Möglichkeit der peroralen Applikation hoher Dosen mit besserem Geschmack, sowie der vereinfachten Herstellung von peroralen Darreichungsformen der R-bzw. S-Thioctsäure mit verbesserter Bioverfügbarkeit.

Eine Verbesserung von Geschmack und Verträglichkeit kann auch erfolgen durch Umhüllung von Granulat, das Thioctsäure oder Salze der Thioctsäure mit physiologisch verträglichen Salzbildnern enthalt. Hierbei werden Überzüge verwendet, die sich erst im Magensaft oder Intestinaltrakt auflösen. Zur Herstellung der Überzüge können beispielsweise verwendet werden: Copolymerisate von Dimethylaminoacrylsäure und neutralen Methacrylsäureestern, die in Wasser und Speichel unlöslich sind, im sauren Bereich aber löslich (zum Beispiel Eudragt®E; Hersteller: Röhm GmbH). Man kann aber auch magensaftresistente Lacke einsetzen wie zum Beispiel Hydroxypropylmethylcellulosephthalat oder -acetatsuccinat; Stärke-, sowie Polyvinylacetatphthalat; Carboxymethylcellulose; Polyvinylacetat; Methylcellulose-phthalat, Methylcellulose-succinat, Methylcellulose phthalatsuccinat sowie Methylcellulose-phthalsäurehalbester; Zein, Ethylcellulose sowie Ethylcellulose-succinat; Schellack; Gluten; Ethylcarboxyethylcellulose; Ethacrylat-Maleinsäureanhydrid-Copolymer; Maleinsäureanhydrid-Vinylmethylether-Copolymer; Styrol-Maleinsäure-Copolymerisate; 2-Ethylhexyl-acrylat-maleinsäureanhydrid; Crotonsäure-Vinylacetat- Copolymer; Glutaminsäure/Glutaminsäureester-Copolymer; Carboxymethylcelluloseglycerinmonooctanoat; Celluloseacetatsuccinat; Polyarginin; Fette, Wachse, Fettalkohole;Anionische Polymerisate aus Methacrylsäure und Methacrylsäureestern (Eudragit®L, Eudragit®S); Copolymerisate aus Acryl- und Methacrylsäureestern mit einem geringen Gehalt an Ammoniumgruppen (Eudragit®RS), sowie Copolymere aus Acryl- und Methacrylsäureestern und Trimethylammoniummethacrylat (Eudragit®RL), Copolymerisat aus Acrylsäureäthyl- und Methacrylsäure-methylestern 70:30 (Eudragit®NE 30 D), Copolymerisat aus Acrylsäure, Methacrylsäure sowie deren Estern (Verhältnis der freien Carboxylgruppen zu den Estergruppen z.B.: 1:1) (Eudragit®L 30 D).

Die genannten Substanzen können zusätzlich übliche Weichmacher (z.B. Dibutylsebacat, Citronen- und Weinsäureester, Glycerin und Glycerinester, Phthalsäureester und ähnliche Stoffe) enthalten, außerdem den Zusatz wasserlöslicher Substanzen wie Polyethylenglykole, Polyvinylpyrrolidon, Copolymerisat aus Polyvinylpyrrolidon und Polyvinylacetat, Hydroxypropylcellulose, Hydroxypropylmethylcellulose. Auch der Zusatz vom Feststoffen wie Talkum und/oder Magnesiumstearat in der Umhüllung ist möglich.

Die Umhüllung erfolgt durch Aufsprühen von Lösungen in organischen Lösungsmitteln oder Wasser, wobei noch weitere Hilfsstoffe zur Optimierung deren Verarbeitbarkeit zugesetzt sein können, wie z.B. oberflächenaktive Substanzen, Pigmente.

Das Aufsprühen erfolgt z.B. im Dragierkessel oder in perforierten Kesseln, oder im Luftsuspensionsverfahren (z.B. Glatt Wirbelschichtanlage WLSD 5).

Die Umhüllung kann auch im Koazervationsverfahren erfolgen, wobei sogenannte Mikrokapseln gebildet werden. Die Umhüllung kann auch durch Koagulation wässriger Dispersionen der zuvor genannten Substanzen erfolgen, indem der Wirkstoff mit der Dispersion vermischt und das Wasser durch Trocknen entfernt wird.

Auf das Granulat sollen 0.0125 - 2 Gewichtsteile Lacktrockensubstanz, bezogen auf ein Gewichtsteil Thioctsäure aufgetragen werden. Darüber hinaus ist es möglich, das Thioctsäure-Granulat mit Xanthan und/oder Maltodextrin und Fumarsäure zu umhüllen, wie in DE 34 40 288 beschrieben. Als Granulatpartikel gelten unregelmäßig geformte oder regelmäßig geformte (beispielsweise sphärische oder zylindrische) Körper, die einen Durchmesser von 0,05 bis 3 mm aufweisen.

Falls es sich bei der erfindungsgemäßen Form um ein Brausegranulat oder eine Brausetablette handelt, enthält diese zusätzlich eine übliche physiologisch unbedenkliche Brausemischung. Die Menge dieser Brausemischung, bezogen auf einen Gewichtsteil Thioctsäure, beträgt 0,05 bis 30 Gewichtsteile. Eine solche besteht aus einer Substanz, die in Gegenwart einer Säure in wässrigem beziehungsweise alkoholischem Milieu Kohlendioxid entwickelt (CO₂-Lieferer) und einer physiologisch verträglichen organischen Säure.

Als CO₂-liefernde Substanzen kommen beispielsweise in Frage: Carbonate oder Hydrogencarbonate des Natriums, Kaliums, Magnesiums oder Calciums, im Falle von Magnesium und Calcium vorzugsweise die neutralen Carbonate. Auch Mischungen dieser Carbonate sind möglich. Als organische Säuren, die aus solchen Carbonaten Kohlendioxid freisetzen, kommen beispielsweise in Frage: organische gesättigte oder ungesättigte Di- und Tricarbonsäuren mit 2 bis 8, vorzugsweise 2 bis 6 C-Atomen, die auch eine, zwei, drei oder vier, vorzugsweise eine oder zwei Hydroxygruppen enthalten können. Beispiele hierfür sind: Citronensäure, Weinsäure, Adipinsäure, Maleinsäure, Fumarsäure. Ebenfalls ist Alginsäure verwendbar. Auch Gemische der genannten Säuren sind möglich.
Auf einen Gewichtsteil Thioctsäure werden beispielsweise 0.02 - 18 Gewichtsteile der CO₂ -liefernden Komponente der Brausemischung und 0.03-12 Gewichtsteile der zugehörigen Säurekomponente verwendet. Bei der Herstellung der Brausetabletten kann zum Beispiel die komplette Brausemischung als solche nach der Granulierungsstufe zugesetzt werden. Es ist aber auch möglich beziehungsweise bevorzugt, die Komponente der Brausemischung, die das Kohlendioxid liefert, vor der Granulierung zuzusetzen und die entsprechende zugehörige Säurekomponente dann später dem trockenen Granulat zusammen mit beispielsweise dem Gleitmittel, den Geschmack-, Süß-und Aroma-stoffen sowie gegebenenfalls den restlichen Füll-, Binde- und Sprengmitteln beizumischen.

Zur Herstellung des Granulates oder der Kau-oder Brausetabletten werden übliche Verfahren angewendet, wie sie in der pharmazeutisch-technologischen Literatur beschrieben sind (beispielsweise in dem Standardwerk Sucker, Fuchs, Speiser, Pharmazeutische Technologie, Thieme Verlag, Stuttgart). Die Herstellung (alle Schritte außer Trocknung) erfolgt zum Beispiel bei Temperaturen zwischen 10 C und 80 C, vorzugsweise 18 C bis 50 C insbesondere 20 C bis 30 C. Die Trocknung des Granulats erfolgt beispielsweise zwischen 30 C und 80 C vorzugsweise 40 C bis 70 C. Bei der Herstellung können alle pharmazeutisch gebräuchlichen Bindemittel wie Cellulosederivate (zum Beispiel Ethylcellulose, Hydroxyethylcellulose, Carboxymethylcellulose, Methylcellulose, Hydroxypropylmethylcellulose), Gelatine, Stärke, Polyglycole (mittlere Molmasse 1000 - 3500 Dalton), Polyvinylalkohole, Polyvinylpyrrolidon, Polyacrylsäure, Vinylpyrrolidon-Vinylacetat-Copolymerisat, Alginate, Saccharose oder Glucose, Polysaccharide, wie zum Beispiel natürliche Gummisorten, wie zum Beispiel Gummi Arabicum, Tragant, Pektin, Cyclodextrin, Guar-Gummi in Mengen von 1 -30 Gewichts%, vorzugsweise 5 - 20 Gewichts%, insbesondere 10 - 15 Gewichts% bezogen auf die Granulatmenge eingesetzt werden (Konzentration der wässrigen Bindemittellösungen 2 -30 Gewichts% vorzugsweise 5 - 15 % Gewichts%). Dabei können auch gleichzeitig verschiedene Bindemittel, zum Beispiel unterschiedliche Cellulosederivate nebeneinander, eingesetzt werden. Die Bindemittel können in die trockene Pulvermischung eingearbeitet werden oder in der Granulierflüssigkeit gelöst oder dispergiert eingebracht werden.

Auch die Kombination von trocken vorgelegtem und gelöstem beziehungsweise dispergiertem Bindemittel ist geeignet. Falls erforderlich, wird das Granulat mit Füll-, Binde-, Spreng-, Netz-, Fließhilfsmittel, Schmiermittel und/oder Gegenklebemittel versetzt.

Als Füllmittel können beispielsweise eingesetzt werden: Cellulose, Cellulosederivate, Saccharose, Lactose, Glucose, Fructose, Calciumphosphate, Calciumsulfate, Calciumcarbonate, Stärke, modifizierte Stärke, Zuckeralkohole wie Sorbitol oder Mannitol.

Als Sprengmittel können beispielsweise eingesetzt werden: Stärke, modifizierte Stärke (beispielsweise Natrium-Stärkeglycolat, Starch 1500), Cellulose, Cellulosederivate, Alginate, quervernetztes Polyvinylpyrrolidon oder quervernetzte Carboxymethylcellulose (Ac-Di-Sol/FMC).

Zur Verbesserung des Suspendierung in Flüssigkeiten kann das Granulat oder die Brausetablette Netzmittel enthalten. Als Beispiele seien genannt: Alkaliseifen, wie Alkalisalze höherer Fettsäuren (z.B. Na-palmitat, Na-stearat oder deren Derivate (z.B. Narizinolatschwefelsäureester); sulfurierte Verbindungen oder sulfonierte Verbindungen, die durch Umsetzung von höheren Fettalkoholen mit Schwefelsäure oder Chlorsulfonsäure entstehen und z.B. als Natriumsalze eingesetzt werden (z.B. Natriumlaurylsulfat, Natriumcetylsulfat, Natriumstearylsulfat, Natriumcetylsulfonat); Salze der Gallensäuren; Saponine; quartäre Ammoniumverbindungen; Partialfettsäureester des Sorbitans; Partialfettsäureester und Fettsäureester des Polyoxyethylensorbitans; Sorbitolether des Polyoxyethylens; Fettalkoholether des Polyoxyethylens; Fettsäureester der Saccharose; Fettsäureester des Polyglycerols; Proteine; Lecithine. Als Fließhilfsmittel eignen sich beispielsweise: Kolloidales Siliciumdioxid, Talkum oder Magnesiumstearat. Als Schmiermittel können beispielsweise eingesetzt werden: Magnesiumstearat, Calciumstearat, D,L-Leucin, Talkum, Stearinsäure, Polyglycole (mittlere Molmasse 3000 - 35000), Fettalkohole oder Wachse. Als Gegenklebemittel sind beispielsweise einsetzbar: Stärke, Talkum, Magnesiumstearat, Calciumstearat oder D,L-Leucin.

Das Granulat, die Kau-, Trink-oder Brausetablette enthalten beispielsweise 100 mg bis maximal 3 g Thioctsäure. Thioctsäure ist im allgemeinen in einer solchen Menge vorhanden, daß es 10 - 80 Gewichtsprozent ausmacht. Es empfiehlt sich darüberhinaus, Geschmack-, Süß- und/oder Aromastoffe zuzumischen. Als Aromastoffe kommen zum Beispiel folgende Pulveraromen in Frage: Ananas, Apfel, Aprikose, Himbeere, Kirsche, Kola, Orange, Passionsfrucht, Zitrone Grapefruit, Vanille, Schokolade. Das Granulat soll 0.05 - 0.2 Gewichtsteile Aroma, bezogen auf 1 Gewichtsteil Thioctsäure enthalten.

Als Süßungsmittel können folgende Stoffe eingesetzt werden: Saccharin und sein Natriumsalz, Cyclaminsäure und ihr Natriumsalz, Ammoniumglycyrrhizinat, Fructose, Xylit, Sorbit, Mannit, Aspartam, Acesulfam-K. Besonders bevorzugt ist eine Mischung aus 1 Teil Saccharin-Natrium und 10 Teilen Natriumcyclamat (jeweils Gewichtsteile). Das Granulat soll 0.003-12 Gewichtsteile Süßungsmittel, bezogen auf 1 Gewichtsteil Thioctsäure oder Dihydroliponsäure enthalten.

### Beispiel

### Trinktablette mit 600 mg Thioctsäure

In 3 l gereinigtem Wasser werden 60 g Natriumalginat gelöst, 300 g Thioctsäure eingerührt und homogenisiert. Diese Suspension wird mit Hilfe eines Wirbelschichtsprühgranulats auf eine Mischung, bestehend aus 40 g Hochdispersem Siliciumdioxid und 250 g vernetztem Polyvidon, gesprüht. Das so entstandene Granulat wird gesiebt und erneut im Wirbelschichtsprüchgranulator mit einer Lösung, bestehend aus 96 g Calciumchlorid in 900 ml gereinigtem Wasser, besprüht.
Ds so entstandene Produkt wird gesiebt, mit 5 g Magnesiumstearat und 4 g Hochdispersem Siliciumdioxid vermischt und zu Tabletten vom Gewicht 1510 mg verpreßt. Eine Tablette enthält 600 mg Thioctsäure. Die Tablette wird zur Anwendung in Wasser zerfallen gelassen.

## Patentansprüche

1. Granulat, Kau-, Trink- oder Brausetabletten, bei denen Thioctsäure oder deren Salze in einem Granulat vorliegen, das mit physiologisch verträglichen Überzügen, die in Wasser und Speichel unlöslich, aber im Magen- oder Intestinalsaft löslich sind, versehen ist.

## Claims

1. Granulate, chewable, drinkable or effervescent tablets wherein thioctic acid or its salts are present in a granulate that is provided with physiologically acceptable coatings which are insoluble in water and saliva but soluble in the gastric or intestinal juice.

## Revendications

1. Granulés, comprimés à mâcher, à boire ou effervescents où l'acide thioctique ou ses sels est ou sont présents dans des granulés qui sont munis d'enrobages physiologiquement acceptables qui sont insolubles dans l'eau et la salive mais solubles dans le suc gastrique ou intestinal.
